(19) Europäisches Patentamt
European Patent Office
Office européen des brevets

(11) EP 0 722 710 B1

(12) **FASCICULE DE BREVET EUROPEEN**

(45) Date de publication et mention de la délivrance du brevet: **06.10.1999 Bulletin 1999/40**

(51) Int. Cl.[6]: **A61K 7/13**

(21) Numéro de dépôt: **96400013.7**

(22) Date de dépôt: **03.01.1996**

(54) **Composition de teinture d'oxydation des fibres kératiniques et procédé de teinture mettant en oeuvre cette composition**

Oxidationsfärbemittel für keratinische Fasern sowie Färbungsverfahren unter Verwendung dieser Zusammensetzung

Keratinous fiber oxidation dyeing composition and the dyeing process using this composition

(84) Etats contractants désignés:
**AT BE CH DE DK ES FR GB GR IE IT LI LU MC NL PT SE**

(30) Priorité: **20.01.1995 FR 9500661**

(43) Date de publication de la demande: **24.07.1996 Bulletin 1996/30**

(73) Titulaire: **L'OREAL**
**75008 Paris (FR)**

(72) Inventeurs:
- **Audousset, Marie-Pascale**
  **F-92600 Asnieres (FR)**
- **Cotteret, Jean**
  **F-78480 Verneuil sur Seine (FR)**

(74) Mandataire:
**Andral, Christophe André Louis**
**L'OREAL-DPI**
**6 rue Bertrand Sincholle**
**92585 Clichy Cedex (FR)**

(56) Documents cités:
**EP-A- 0 428 441**
**EP-A- 0 465 340**
**DE-A- 3 031 709**
**DE-A- 3 743 769**
**DE-A- 3 942 294**

EP 0 722 710 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention a pour objet une composition prête à l'emploi pour la teinture d'oxydation des fibres kératiniques, en particulier des fibres kératiniques humaines telles que les cheveux, comprenant, dans un milieu alcalin, un précurseur de colorant d'oxydation en association avec le 4-hydroxyindole, un coupleur benzénique et un agent oxydant, ainsi que le procédé de teinture mettant en oeuvre cette composition. Elle concerne également un kit de coloration pour la préparation d'une telle composition prête à l'emploi.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés indoliques comme le 4-hydroxyindole. La demande de brevet DE 39 42 294 divulgue des procédés de teinture par oxydation des fibres kératiniques utilisant ces bases d'oxydation en association avec ces coupleurs.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

On connait dans la demande de brevet européen EP-A-0465340, des compositions de teinture des fibres kératiniques à pH acide renfermant une base d'oxydation, le 4-hydroxyindole à titre de coupleur et éventuellement des coupleurs additionnels classiques, utilisées pour obtenir des colorations naturelles et cendrées.

On connait également dans la demande de brevet allemand DE 3 031 709, des compositions alcalines pour la teinture d'oxydation des fibres kératiniques renfermant une base d'oxydation , le 4-hydroxyindole à titre de coupleur et éventuellement des coupleurs additionnels classiques tels que la résorcine. Ces compositions ne sont cependant pas entièrement satisfaisantes, notamment au niveau de la sélectivité des colorations obtenues. D'autre part, la demande de brevet allemand DE 3 743 769 décrit une composition alcaline de teinture d'oxydation des fibres kératiniques renfermant de la 2,3-diméthyl paraphénylènediamine à titre de base d'oxydation en association avec un coupleur pouvant être notamment le 4-hydroxyindole. Ces compositions ne sont cependant pas entièrement satisfaisantes, notamment au niveau de la tenue des colorations obtenues vis à vis des déformations permanentes et des lavages.

La présente invention vise à proposer de nouvelles compositions pour la coloration d'oxydation des fibres kératiniques et en particulier des fibres kératiniques humaines telles que les cheveux qui présentent de très bonnes propriétés tinctoriales.

Ainsi, la demanderesse vient en effet de découvrir qu'il est possible d'obtenir de nouvelles teintures, qui engendrent des colorations résistantes, intenses et peu sélectives, en associant :

- au moins une base d'oxydation convenablement sélectionnée et telle que définie ci-après,
- du 4-hydroxyindole à titre de premier coupleur,
- au moins un coupleur benzénique additionnel convenablement sélectionné et tel que défini ci-après,
- au moins un agent oxydant,
  le pH de la composition tinctoriale résultante étant supérieur ou égal à 7.

Cette découverte est à la base de la présente invention.

L'invention a donc pour premier objet une composition prête à l'emploi pour la teinture d'oxydation des fibres kératiniques et en particulier des fibres kératiniques humaines telles que les cheveux, présentant un pH supérieur ou égal à 7, et contenant, dans un milieu approprié pour la teinture, au moins un agent oxydant, et au moins une association d'au moins une base d'oxydation, de 4-hydroxyindole à titre de coupleur, et d'au moins un coupleur benzénique additionnel, caractérisée par le fait que ladite association est choisie parmi les associations a) à h) suivantes :

**a)** para-aminophénol, 4-hydroxyindole, 5-N-(β-hydroxyéthyl)amino 2-méthyl phénol ;

**b)** paraphénylènediamine, 4-hydroxyindole, 2-méthyl 5-aminophénol ;

**c)** 3-méthyl 4-amino phénol, 4-hydroxyindole, dichlorhydrate de 2,4-diamino 1-(β-hydroxyéthyloxy) benzène ;

**d)** dichlorhydrate de 2,6-diméthyl paraphénylènediamine, 4-hydroxyindole, dichlorhydrate de 2-amino 4-N-(β-hydroxyéthyl)amino anisole ;

**e)** dichlorhydrate de 2-β-hydroxyéthyl paraphénylènediamine, 4-hydroxyindole, méta-aminophénol ;

**f)** dichlorhydrate de 3,4-diamino pyrazole, 4-hydroxyindole, 5-N-(β-hydroxyéthyl)amino 2-méthyl phénol ;

**g)** paraphénylènediamine, 4-hydroxyindole, méta-aminophénol ;

**h)** dichlorhydrate de 2,3-diméthyl paraphénylènediamine, 4-hydroxyindole, 2-méthyl 5-aminophénol.

Les colorations obtenues avec les compositions tinctoriales selon l'invention sont faiblement sélectives et présentent par ailleurs une bonne puissance tinctoriale et d'excellentes propriétés de résistances à la fois vis à vis des agents atmosphériques tels que la lumière et les intempéries et vis à vis de la transpiration et des différents traitements que peuvent subir les cheveux (lavages, déformations permanentes).

L'invention a également pour objet un procédé de teinture d'oxydation des fibres kératiniques mettant en oeuvre cette composition tinctoriale.

Les sels d'addition avec un acide utilisables dans le cadre des compositions tinctoriales de l'invention sont notamment choisis parmi les chlorhydrates, les bromhydrates, les sulfates et les tartrates.

La ou les bases d'oxydation conformes à l'invention représentent de préférence de 0,0005 à 10 % en poids environ du poids total de la composition tinctoriale et encore plus préférentiellement de 0,01 à 5 % en poids environ.

Le 4-hydroxyindole représente de préférence de 0,0001 à 3,5 % en poids environ du poids total de la composition tinctoriale et encore plus préférentiellement de 0,005 à 1 % en poids environ.

Le ou les coupleurs benzéniques additionnels conformes à l'invention représentent de préférence de 0,0001 à 5 % en poids environ du poids total de la composition tinctoriale et encore plus préférentiellement de 0,001 à 3.% en poids environ.

Le pH de la composition tinctoriale telle que définie ci-dessus peut varier entre 7 et 12 et de préférence entre 8 et 11 et peut être ajusté à la valeur désirée au moyen d'agents acidifiants ou alcalinisants habituellement utilisés en teinture des fibres kératiniques.

Parmi les agents acidifiants on peut citer, à titre d'exemple, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (IV) suivante :

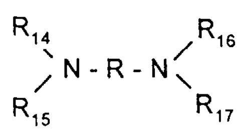

(IV)

dans laquelle R est un reste propylène éventuellement substitué par un groupement hydroxyle ou un radical alkyle en $C_1$-$C_4$ ; $R_{14}$, $R_{15}$, $R_{16}$ et $R_{17}$ identiques ou différents, représentent un atome d'hydrogène, un radical alkyle en $C_1$-$C_4$ ou hydroxyalkyle en $C_1$-$C_4$.

L'agent oxydant présent dans la composition tinctoriale est choisi parmi les agents oxydants classiquement utilisés en coloration d'oxydation et de préférence parmi le peroxyde d'hydrogène, le peroxyde d'urée, les bromates de métaux alcalins, les persels tels que les perborates et persulfates. Le peroxyde d'hydrogène est particulièrement préféré.

La composition tinctoriale conforme à l'invention peut encore contenir, en plus des colorants définis ci-dessus, d'autres coupleurs et/ou des colorants directs, notamment pour modifier les nuances ou les enrichir en reflets.

Le milieu approprié pour la teinture (ou support) de la composition tinctoriale est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en $C_1$-$C_4$, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids

environ par rapport au poids total de la composition tinctoriale, et encore plus préférentiellement entre 5 et 30 % en poids environ.

Les compositions tinctoriales conformes à l'invention peuvent également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement, des agents filmogènes, des agents conservateurs, des agents opacifiants.

Les compositions tinctoriales conformes à l'invention peuvent se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres la composition tinctoriale telle que définie précédemment, et on laisse poser pendant 3 à 40 minutes environ, de préférence 5 à 30 minutes environ, après quoi on rince, on lave éventuellement au shampooing, on rince à nouveau et on sèche.

Selon une forme de réalisation préférée, le procédé comporte une étape préliminaire consistant à stocker sous forme séparée, d'une part, une composition (A) comprenant, dans un milieu approprié pour la teinture, au moins une association choisie parmi les associations a) à h) telles que définies précédemment et, d'autre part, une composition (B) renfermant, dans un milieu approprié pour la teinture, au moins un agent oxydant tel que défini précédemment, et à procéder à leur mélange au moment de l'emploi avant d'appliquer ce mélange sur les fibres kératiniques, le pH des compositions (A) et (B) étant tel qu'après mélange de 10 à 90 % de la composition (A) avec 90 à 10 % de la composition (B), le pH du mélange résultant soit supérieur ou égal à 7.

La pH des compositions (A) et (B) peut être ajusté à la valeur désirée au moyen d'agents alcalinisants ou acidifiants classiques et tels que définis précédemment.

Un autre objet de l'invention est un dispositif à plusieurs compartiments ou "kit" de teinture ou tout autre système de conditionnement à plusieurs compartiments dont un premier compartiment renferme la composition (A) telle que définie ci-dessus et un second compartiment renferme la composition oxydante (B) telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

**EXEMPLES**

**<u>EXEMPLES 1 à 6</u>**

On a préparé les compositions 1 (A) à 6 (A), conformes à l'invention, suivantes (teneurs en grammes) :

| COMPOSITION | 1 (A) | 2 (A) | 3 (A) | 4 (A) | 5 (A) | 6 (A) |
|---|---|---|---|---|---|---|
| Para-aminophénol | 1 | | | | | |
| Paraphénylènediamine | | 0,3 | | | | |
| 3-méthyl 4-aminophénol | | | 0,3 | | | |
| Dichlorhydrate de 2,6-diméthyl paraphénylènediamine | | | | 0,5 | | |
| Dichlorhydrate de 2-β-hydroxyéthyl paraphénylènediamine | | | | | 0,3 | |
| Dichlorhydrate de 3,4-diamino pyrazole | | | | | | 0,3 |
| 4-hydroxyindole | 0,5 | 0,15 | 0,1 | 0,1 | 0,1 | 0,2 |
| 5-N-(β-hydroxyéthylamino) 2-méthyl phénol | 0,5 | | | | | 0,1 |
| 2-méthyl 5-aminophénol | | 0,2 | | | | |
| Dichlorhydrate de 2,4-diamino 1-(β-hydroxyéthyloxy) benzène | | | 0,2 | | | |
| Dichlorhydrate de 2-amino 4-N-(β-hydroxyéthylamino) anisole | | | | 0,4 | | |
| Méta-aminophénol | | | | | 0,2 | |
| Support de teinture commun (*) | (*) | (*) | (*) | (*) | (*) | (*) |
| Eau q.s.p. | 100 g | 100 g | 100 g | 100 g | 100 g | 100 g |

(*) support de teinture commun :

| | | |
|---|---|---|
| - Alcool oléique polyglycérolé à 2 moles de glycérol | 4,0 | g |
| - Alcool oléique polyglycérolé à 4 moles de glycérol, à 78 % de matières actives (M.A.) | 5,69 | g M.A. |
| - Acide oléique | 3,0 | g |
| - Amine oléique à 2 moles d'oxyde d'éthylène vendue sous la dénomination commerciale ETHOMEEN O12 par la société AKZO | 7,0 | g |
| - Laurylamino succinamate de diéthylaminopropyle, sel de sodium, à 55 % de M.A. | 3,0 | g M.A. |
| - Alcool oléique | 5,0 | g |
| - Diéthanolamide d'acide oléique | 12,0 | g |
| - Propylèneglycol | 3,5 | g |
| - Alcool éthylique | 7,0 | g |
| - Dipropylèneglycol | 0,5 | g |
| - Monométhyléther de propylèneglycol | 9,0 | g |
| - Métabisulfite de sodium en solution aqueuse, à 35 % de M.A. | 0,455 | g M.A. |
| - Acétate d'ammonium | 0,8 | g |
| - Antioxydant, séquestrant | q.s. | |
| - Parfum, conservateur | q.s. | |
| - Ammoniaque à 20 % de $NH_3$ | 10,0 | g |

Au moment de l'emploi, on a mélangé chacune de ces compositions 1 (A) à 6 (A) avec une quantité égale d'une composition (B) constituée par une solution d'eau oxygénée à 20 volumes (6 % en poids).

Chaque composition résultante (composition prête à l'emploi conforme à l'invention, de pH $\geq$ 7) a été appliquée pendant 30 minutes sur des mèches de cheveux gris à 90 % de blancs, naturels ou permanentés. Les mèches de cheveux ont ensuite été rincées, lavées avec un shampooing standard puis séchées.

Les mèches de cheveux ont été teintes dans les nuances figurant dans le tableau ci-dessous :

| EXEMPLE [COMPOSITION] | NUANCE SUR CHEVEUX NATURELS | NUANCE SUR CHEVEUX PERMANENTES |
|---|---|---|
| 1 [1 (A)] | blond cuivré irisé | blond cuivré irisé rouge |
| 2 [2 (A)] | blond violine | blond foncé violine |
| 3 [3 (A)] | blond clair beige irisé | blond irisé |
| 4 [4 (A)] | bleu cendré | bleu profond |

(suite)

| EXEMPLE [COMPOSITION] | NUANCE SUR CHEVEUX NATURELS | NUANCE SUR CHEVEUX PERMANENTES |
|---|---|---|
| 5 [5 (A)] | violine cendré léger | violine cendré |
| 6 [6 (A)] | rose fuchsia | rose fuchsia intense |

**EXEMPLES 7 ET 8 COMPARATIFS**

On a préparé les compositions 7 (A) et 8 (A) suivantes :

Composition 7 (A) conforme à l'invention :

| | | |
|---|---|---|
| - Paraphénylènediamine | 0,4 | g |
| - 4-hydroxyindole | 0,1 | g |
| - Méta-aminophénol | 0,3 | g |
| - Support de teinture commun défini aux exemples 1 à 6 | (*) | g |
| - Eau q.s.p. | 100 | g |

Composition 8 (A) ne faisant pas partie de l'invention :

| | | |
|---|---|---|
| - Paraphénylènediamine | 0,4 | g |
| - 4-hydroxyindole | 0,1 | g |
| - Résorcine (coupleur additionnel) | 0,3 | g |
| - Support de teinture commun défini aux exemples 1 à 6 | (*) | g |
| - Eau q.s.p. | 100 | g |

Au moment de l'emploi, on a mélangé chaque composition 7 (A) et 8 (A) avec une quantité égale d'une composition (B) constituée par une solution d'eau oxygénée à 20 volumes (6 % en poids).

Chaque composition résultante présentait un pH $\geq$ 7 et a été appliquée pendant 30 minutes, d'une part sur des mèches de cheveux gris naturels à 90 % de blancs (mèche n°1 de cheveux non sensibilisés) et d'autre part sur une mèche de ces mêmes cheveux gris à 90 % de blancs mais ayant subi une permanente (mèche n° 2 de cheveux sensibilisés). Les mèches de cheveux ont ensuite été rincées, lavées avec un shampooing standard puis séchées.

La couleur des mèches a ensuite été évaluée dans le système MUNSELL au moyen d'un colorimètre CM 2002 MINOLTA.

Selon la notation MUNSELL, une couleur est définie par l'expression **H V / C** dans laquelle les trois paramètres désignent respectivement la teinte ou Hue (**H**), l'intensité ou Value (**V**) et la pureté ou Chromaticité (**C**), la barre oblique de cette expression est simplement une convention et n'indique pas un ratio.

La différence de couleur entre deux mèches est calculée en appliquant la formule de NICKERSON : $\Delta E = 0{,}4\ Co\Delta H + 6\Delta V + 3\ \Delta C$ , telle que décrite par exemple dans "Couleur, Industrie et Technique" ; pages 14-17 ;

vol. n° 5 ; 1978.

Dans cette formule, ΔE représente la différence de couleur entre deux mèches, ΔH, ΔV et ΔC représentent la variation en valeur absolue des paramètres H, V et C et Co représente la pureté de la mèche par rapport à laquelle on désire évaluer la différence de couleur.

Les résultats sont donnés dans le tableau ci-dessous :

| EXEMPLE [COMPOSITION] | Couleur sur cheveux naturels | Couleur sur cheveux permanentés | Différence de couleur (sélectivité) | | | |
|---|---|---|---|---|---|---|
| | | | ΔH | ΔV | ΔC | ΔE |
| 7 [7 (A)] | 3,6 R 2,5 / 1,0 | 9,4 RP 2,5 / 0,9 | 4,2 | 0 | 0,1 | **1,98** |
| 8 [8 (A)] | 6,5 YR 3,1 / 1,3 | 9,3 R 2,5 / 0,9 | 7,2 | 0,6 | 0,4 | **8,5** |

Ces résultats montrent que la composition de l'exemple 7 conforme à l'invention conduit à une coloration non sélective, ce qui n'est pas le cas pour la composition de l'exemple 8 ne faisant pas partie de l'invention et renfermant une association de paraphénylènediamine, de 4-hydroxyindole et de résorcine comme décrit par exemple dans la demande de brevet allemand DE 3 031 709.

**EXEMPLES 9 ET 10 COMPARATIFS**

On a préparé les compositions 9 (A) et 10 (A) suivantes :

Composition 9 (A) conforme à l'invention :

| | | |
|---|---|---|
| - Dichlorhydrate de 2,3-diméthyl paraphénylènediamine | 0,4 | g |
| - 4-hydroxyindole | 0,2 | g |
| - 2-méthyl 5-aminophénol | 0,2 | g |
| - Support de teinture commun défini aux exemples 1 à 6 | (*) | g |
| - Eau q.s.p. | 100 | g |

Composition 10 (A) ne faisant pas partie de l'invention :

| | | |
|---|---|---|
| - Dichlorhydrate de 2,3-diméthyl paraphénylènediamine | 0,4 | g |
| - 4-hydroxyindole | 0,2 | g |
| - α-naphtol (coupleur additionnel) | 0,2 | g |
| - Support de teinture commun défini aux exemples 1 à 6 | (*) | g |
| - Eau q.s.p. | 100 | g |

Au moment de l'emploi, on a mélangé chaque composition 9 (A) et 10 (A) avec une quantité égale d'une composition (B) constituée par une solution d'eau oxygénée à 20 volumes (6 % en poids).

Chaque composition résultante présentait un pH ≥ 7 et a été appliquée pendant 30 minutes, sur des mèches de cheveux gris permanentés à 90 % de blancs. Les mèches de cheveux ont ensuite été rincées, lavées avec un shampooing standard puis séchées.

La couleur des mèches a ensuite été évaluée dans le système MUNSELL au moyen d'un colorimètre CM 2002 MINOLTA.

Les mèches de cheveux ainsi teintes ont ensuite été soumises à un test de résistance aux shampooings (machine Ahiba-Texomat) :

Les mèches de cheveux ont été placées dans un panier que l'on a immergé dans une solution d'un shampooing standard à 37°C. Le panier a été soumis à un mouvement de va-et-vient vertical de fréquence variable ainsi qu'à un mouvement de rotation qui reproduisent l'action d'un frottement manuel, ce qui engendre la formation de mousse.

Après 3 minutes d'épreuve, on a retiré les mèches que l'on a rincées puis séchées. Les mèches teintes ont été soumises à 4 épreuves de shampooing consécutives.

La couleur des mèches a été ensuite évaluée à nouveau dans le système MUNSELL au moyen d'un colorimètre CM 2002 MINOLTA.

La dégradation de la coloration due aux shampooings a été calculée en appliquant la formule de NICKERSON telle que décrite précédemment aux exemples 7 et 8.

Les résultats sont donnés dans le tableau ci-dessous :

| EXEMPLE [COMPOSITION] | Couleur des cheveux avant les shampooings | Couleur des cheveux après les shampooings | Dégradation de la couleur | | | |
|---|---|---|---|---|---|---|
| | | | ΔH | ΔV | ΔC | ΔE |
| **9 [9 (A)]** | 7,1 P 3,1 / 1,4 | 9,0 P 3,7 / 1,2 | 1,9 | 0,6 | 0,2 | **5,3** |
| **10 [10 (A)]** | 1,1 RP 3,2 / 2,0 | 7,8 RP 3,9 / 1,5 | 6,7 | 0,7 | 0,5 | **11,1** |

Ces résultats montrent que la composition de l'exemple 9 conforme à l'invention conduit à une coloration résistant beaucoup mieux aux shampooings que la composition de l'exemple 10 ne faisant pas partie de l'invention et telle que décrite par exemple dans la demande de brevet allemand DE 3 743 769.

## Revendications

**1.** Composition prête à l'emploi pour la teinture d'oxydation des fibres kératiniques et en particulier des fibres kératiniques humaines telles que les cheveux, présentant un pH supérieur ou égal à 7, et contenant, dans un milieu approprié pour la teinture, au moins un agent oxydant, et au moins une association d'au moins une base d'oxydation, de 4-hydroxyindole à titre de coupleur, et d'au moins un coupleur benzénique additionnel, caractérisée par le fait que ladite association est choisie parmi les associations a) à h) suivantes :

**a)** para-aminophénol, 4-hydroxyindole, 5-N-(β-hydroxyéthyl)amino 2-méthyl phénol ;
**b)** paraphénylènediamine, 4-hydroxyindole, 2-méthyl 5-aminophénol ;
**c)** 3-méthyl 4-amino phénol, 4-hydroxyindole, dichlorhydrate de 2,4-diamino 1-(β-hydroxyéthyloxy) benzène ;
**d)** dichlorhydrate de 2,6-diméthyl paraphénylènediamine, 4-hydroxyindole, dichlorhydrate de 2-amino 4-N-(β-hydroxyéthyl)amino anisole ;
**e)** dichlorhydrate de 2-β-hydroxyéthyl paraphénylènediamine, 4-hydroxyindole, méta-aminophénol ;
**f)** dichlorhydrate de 3,4-diamino pyrazole, 4-hydroxyindole, 5-N-(β-hydroxyéthyl)amino 2-méthyl phénol ;
**g)** paraphénylènediamine, 4-hydroxyindole, méta-aminophénol ;
**h)** dichlorhydrate de 2,3-diméthyl paraphénylènediamine, 4-hydroxyindole, 2-méthyl 5-aminophénol.

**2.** Composition selon la revendication 1, caractérisée par le fait que la ou les bases d'oxydation représentent de 0, 0005 à 10 % en poids du poids total de la composition tinctoriale, et de préférence de 0,01 à 5 % en poids.

**3.** Composition selon la revendication 1 ou 2, caractérisée par le fait que le 4-hydroxyindole représente de 0,0001 à 3,5 % en poids du poids total de la composition tinctoriale, et de préférence de 0,005 à 1 % en poids.

4. Composition selon l'une quelconque des revendications 1 à 3, caractérisée par le fait que le ou les coupleurs benzéniques additionnels représentent de 0,0001 à 5 % en poids du poids total de la composition tinctoriale, et de préférence de 0,001 à 3 % en poids.

5. Composition selon l'une quelconque des revendications 1 à 4, caractérisée par le fait qu'elle présente un pH compris entre 7 et 12, et de préférence entre 8 et 11.

6. Composition selon l'une quelconque des revendications 1 à 5, caractérisée par le fait que l'agent oxydant est choisi parmi le peroxyde d'hydrogène, le peroxyde d'urée, les bromates de métaux alcalins, les persels tels que les perborates et persulfates.

7. Composition selon l'une quelconque des revendications 1 à 6 caractérisée par le fait qu'elle renferme, en outre, au moins un adjuvant choisi parmi les agents tensio-actifs anioniques, cationiques, non-ioniques, amphotères, zwittérioniques ou leurs mélanges, les polymères anioniques, cationiques, non-ioniques, amphotères, zwittérioniques ou leurs mélanges, les agents épaississants minéraux ou organiques, les agents antioxydants, les agents de pénétration, les agents séquestrants, les parfums, les tampons, les agents dispersants, les agents de conditionnement, les agents filmogènes, les agents conservateurs, les agents opacifiants.

8. Composition selon l'une quelconque des revendications 1 à 7, caractérisée par le fait qu'elle se présente sous des formes diverses, telles que sous forme de liquides, de crèmes, de gels, ou sous toute autre forme appropriée pour réaliser une teinture des fibres kératiniques, et notamment des cheveux humains.

9. Procédé de teinture des fibres kératiniques et en particulier des fibres kératiniques humaines telles que les cheveux caractérisé par le fait que l'on applique sur ces fibres au moins une composition tinctoriale telle que définie dans l'une quelconque des revendications 1 à 8.

10. Procédé selon la revendication 9, caractérisé par le fait qu'il comporte une étape préliminaire consistant à stocker sous forme séparée, d'une part, une composition (A) comprenant, dans un milieu approprié pour la teinture, au moins une association telle que définie à la revendication 1 et, d'autre part, une composition (B) renfermant, dans un milieu approprié pour la teinture, au moins un agent oxydant tel que défini dans la revendication 6, et à procéder à leur mélange au moment de l'emploi avant d'appliquer ce mélange sur les fibres kératiniques, le pH des compositions (A) et (B) étant tel qu'après mélange de 10 à 90 % de la composition (A) avec 90 à 10 % de la composition (B), le pH du mélange résultant soit supérieur ou égal à 7.

11. Dispositif à plusieurs compartiments ou "kit" de teinture, caractérisé par le fait qu'un premier compartiment renferme la composition (A) telle que définie à la revendication 10 et un second compartiment renferme la composition oxydante (B) telle que définie à la revendication 10.

## Claims

1. Ready-to-use composition for the oxidation dyeing of keratin fibres, and in particular human keratin fibres such as the hair, this composition having a pH of greater than or equal to 7 and containing, in a medium which is suitable for dyeing, at least one oxidizing agent and at least one combination of at least one oxidation base, of 4-hydroxyindole as coupler, and of at least one additional benzenic coupler, characterized in that the said combination is chosen from combinations a) to h) below:

   a) para-aminophenol, 4-hydroxyindole, 5-N-(β-hydroxyethyl)amino-2-methylphenol;
   b) para-phenylenediamine, 4-hydroxyindole, 2-methyl-5-aminophenol;
   c) 3-methyl-4-aminophenol, 4-hydroxyindole, 2,4-diamino-1-(β-hydroxyethyloxy)benzene dihydrochloride;
   d) 2,6-dimethyl-para-phenylenediamine dihydrochloride, 4-hydroxyindole, 2-amino-4-N-(β-hydroxyethyl)aminoanisole dihydrochloride;
   e) 2-β-hydroxyethyl-para-phenylenediamine dihydrochloride, 4-hydroxyindole, meta-aminophenol;
   f) 3,4-diaminopyrazole dihydrochloride, 4-hydroxyindole, 5-N-(β-hydroxyethyl)amino-2-methylphenol;
   g) para-phenylenediamine, 4-hydroxyindole, meta-aminophenol;
   h) 2,3-dimethyl-para-phenylenediamine dihydrochloride, 4-hydroxyindole, 2-methyl-5-aminophenol.

2. Composition according to Claim 1, characterized in that the oxidation base(s) represent(s) from 0.0005 to 10% by weight of the total weight of the dye composition, and preferably from 0.01 to 5% by weight.

3. Composition according to Claim 1 or 2, characterized in that the 4-hydroxyindole represents from 0.0001 to 3.5% by weight of the total weight of the dye composition, and preferably from 0.005 to 1% by weight.

4. Composition according to any one of Claims 1 to 3, characterized in that the additional benzenic coupler(s) represent(s) from 0.0001 to 5% by weight of the total weight of the dye composition, and preferably from 0.001 to 3% by weight.

5. Composition according to any one of Claims 1 to 4, characterized in that it has a pH of between 7 and 12 and preferably between 8 and 11.

6. Composition according to any one of Claims 1 to 5, characterized in that the oxidizing agent is chosen from hydrogen peroxide, urea peroxide, alkali metal bromates and persalts such as perborates and persulphates.

7. Composition according to any one of Claims 1 to 6, characterized in that it also contains at least one adjuvant chosen from anionic, cationic, nonionic, amphoteric and zwitterionic surfactants or mixtures thereof, anionic, cationic, nonionic, amphoteric and zwitterionic polymers or mixtures thereof, inorganic or organic thickeners, antioxidants, penetration agents, sequestering agents, fragrances, buffers, dispersing agents, conditioners, film-forming agents, preserving agents and opacifiers.

8. Composition according to any one of Claims 1 to 7, characterized in that it is in various forms, such as in the form of liquids, creams or gels or in any other form which is suitable for dyeing keratin fibres, and in particular human hair.

9. Process for dyeing keratin fibres, and in particular human keratin fibres such as the hair, characterized in that at least one dye composition as defined in any one of Claims 1 to 8 is applied to these fibres.

10. Process according to Claim 9, characterized in that it includes a preliminary step which consists in separately storing, on the one hand, a composition (A) comprising, in a medium which is suitable for dyeing, at least one combination as defined in Claim 1 and, on the other hand, a composition (B) containing, in a medium which is suitable for dyeing, at least one oxidizing agent as defined in Claim 6, and in mixing them together at the time of use, after which this mixture is applied to the keratin fibres, the pH of the compositions (A) and (B) being such that after mixing 10 to 90% of composition (A) with 90 to 10% of composition (B), the pH of the resulting mixture is greater than or equal to 7.

11. Multi-compartment dyeing device or "kit", characterized in that a first compartment contains composition (A) as defined in Claim 10 and a second compartment contains the oxidizing composition (B) as defined in Claim 10.

**Patentansprüche**

1. Gebrauchsfertige Zusammensetzung zum oxidativen Färben von Keratinfasern und insbesondere von menschlichen Keratinfasern, wie dem Haar, die einen pH-Wert von 7 oder darüber aufweist, die in einem zum Färben geeigneten Medium mindestens ein Oxidationsmittel und mindestens eine Kombination aus mindestens einer Oxidationsbase, 4-Hydroxyindol als Kuppler und mindestens einem zusätzlichen Benzol-Kuppler enthält und die dadurch gekennzeichnet ist, daß die Kombination unter den folgenden Kombinationen a) bis h) ausgewählt ist:

   a) p-Aminophenol, 4-Hydroxyindol, 5-N-(β-hydroxyethyl)amino-2-methyl-phenol;
   b) p-Phenylendiamin, 4-Hydroxyindol, 2-Methyl-5-amino-phenol;
   c) 3-Methyl-4-amino-phenol, 4-Hydroxyindol, 2,4-Diamino-1-(β-hydroxyethyloxy)-benzol-Dihydrochlorid;
   d) 2,6-Dimethyl-p-Phenylendiamin-Dihydrochlorid, 4-Hydroxyindol, 2-Amino-4-N-(β-hydroxyethyl)aminoanisol-Dihydrochlorid;
   e) 2-β-Hydroxyethyl-p-phenylendiamin-Dihydrochlorid, 4-Hydroxyindol, m-Aminophenol;
   f) 3,4-Diamino-pyrazol-Dihydrochlorid, 4-Hydroxyindol, 5-N-(β-hydroxyethyl)amino-2-methyl-phenol;
   g) p-Phenylendiamin, 4-Hydroxyindol, m-Aminophenol;
   h) 2,3-Dimethyl-p-Phenylendiamin-Dihydrochlorid, 4-Hydroxyindol, 2-Methyl-5-amino-phenol.

2. Zusammensetzung nach Anspruch 1, dadurch gekennzeichnet, daß die Oxidationsbase oder die Oxidationsbasen 0,0005 bis 10 Gew.-% des Gesamtgewichts der Färbemittelzusammensetzung und vorzugsweise 0,01 bis 5 Gew.-% ausmachen.

3. Zusammensetzung nach Anspruch 1 oder 2, dadurch gekennzeichnet, daß das 4-Hydroxyindol 0,0001 bis 3,5 Gew.-% des Gesamtgewichts der Färbemittelzusammensetzung und vorzugsweise 0,005 bis 1 Gew.-% ausmacht.

4. Zusammensetzung nach einem der Ansprüche 1 bis 3, dadurch gekennzeichnet, daß der oder die zusätzliche(n) Benzol-Kuppler 0,0001 bis 5 Gew.-% des Gesamtgewichts der Färbemittelzusammensetzung und vorzugsweise 0,001 bis 3 Gew.-% ausmachen.

5. Zusammensetzung nach einem der Ansprüche 1 bis 4, dadurch gekennzeichnet, daß sie einen pH-Wert im Bereich von 7 bis 12 und vorzugsweise von 8 bis 11 aufweist.

6. Zusammensetzung nach einem der Ansprüche 1 bis 5, dadurch gekennzeichnet, daß das Oxidationsmittel unter Wasserstoffperoxid, Harnstoffperoxid, Alkalimetallbromaten und Salzen von Persäuren, wie Perboraten und Persulfaten, ausgewählt ist.

7. Zusammensetzung nach einem der Ansprüche 1 bis 6, dadurch gekennzeichnet, daß sie ferner mindestens einen Hilfsstoff enthält, der unter den anionischen, kationischen, nichtionischen, amphoteren, zwitterionischen grenzflächenaktiven Stoffen oder deren Gemischen, anionischen, kationischen, nichtionischen, amphoteren, zwitterionischen Polymeren oder deren Gemischen, anorganischen oder organischen Verdickungsmitteln, Antioxidantien, Penetrationsmitteln, Maskierungsmitteln, Parfums, Puffern, Dispergiermitteln, Konditioniermitteln, Filmbildnern, Konservierungsmitteln und Trübungsmitteln ausgewählt ist.

8. Zusammensetzung nach einem der Ansprüche 1 bis 7, dadurch gekennzeichnet, daß sie in unterschiedlichen Formen vorliegt, wie als Flüssigkeit, Creme, Gel, oder in beliebigen weiteren Formen, die zur Durchführung einer Färbung von Keratinfasern und insbesondere des menschlichen Haares geeignet sind.

9. Verfahren zum Färben von Keratinfasern und insbesondere von menschlichen Keratinfasern, wie dem Haar, dadurch gekennzeichnet, daß auf die Fasern mindestens eine Färbemittelzusammensetzung nach einem der Ansprüche 1 bis 8 aufgetragen wird.

10. Verfahren nach Anspruch 9, dadurch gekennzeichnet, daß es einen vorbereitenden Schritt umfaßt, der darin besteht, getrennt voneinander einerseits eine Zusammensetzung (A), die in einem zum Färben geeigneten Medium mindestens eine Kombination nach Anspruch 1 enthält, und andererseits eine Zusammensetzung (B) aufzubewahren, die in einem zum Färben geeigneten Medium mindestens ein Oxidationsmittel, wie in Anspruch 6 definiert, enthält, und diese bei der Anwendung zu vermischen, bevor das Gemisch auf die Fasern aufgebracht wird, wobei der pH-Wert der Zusammensetzungen (A) und (B) so ist, daß nach dem Mischen von 10 bis 90 % Zusammensetzung (A) und 90 bis 10 % Zusammensetzung (B) der pH-Wert des resultierenden Gemisches bei 7 oder darüber liegt.

11. Vorrichtung mit mehreren Abteilungen oder "Kit" zum Färben, dadurch gekennzeichnet, daß eine erste Abteilung die Zusammensetzung (A) nach Anspruch 10 und eine zweite Abteilung die oxidierende Zusammensetzung (B) nach Anspruch 10 enthält.